# EUROPEAN PATENT APPLICATION

(11) **EP 1 719 540 A2**
(43) Date of publication of application: **08.11.2006**
(21) Application number: 06252394.9
(22) Date of filing: 05.05.2006
(51) Int. Cl.: A61N 1/05, A61N 1/08

(54) **Lead insertion tool**

(30) Priority: 05.05.2005 US 678626 P
(71) Applicant: Alfred E. Mann Foundation for Scientific Research, Santa Clarita, CA 91380-9005 (US)
(72) Inventor: Schulman, Joseph H., Santa Clarita, CA 91387 (US); Zilberman, Yitzhak, Santa Clarita, CA 91321 (US)
(74) Representative: Carter, Stephen John

(57) **Abstract**

An implantation device comprising a hollow tube for locating an electrode of a lead of a lead adjacent to or in contact with a nerve, muscle or organ in living tissue. The device includes an opening extending the length of the device adapted for introducing and releasing a lead from the tube. The distal end of the device includes a sharpened edge for penetrating through tissue and defines a recess adapted to retain the electrode as the device penetrates into tissue. The electrode is released from the device as it is withdrawn from the tissue. The electrode may be formed of metal having shape memory configured to wrap around a neuromuscular pathway.

## Description

### FIELD OF THE INVENTION

Examples of the present invention relate to the placement of a lead electrode adjacent to or in contact with a neuromuscular pathway or organ.

### SUMMARY OF THE INVENTION

According to a first aspect of the present invention there is provided an insertion device configured for subcutaneous insertion of a lead, comprising
a hollow tube having a proximal end and a distal end;
the tube having a longitudinal opening extending from the proximal end to the distal end;
the opening sized to receive at least an electrical lead along the length of the opening; and
the distal end having a sharpened angled edge for penetrating living tissue.

Preferably, the distal end of the device includes a U-shaped portion adapted for receiving an end of the lead.

Preferably, the device is formed of a biocompatible material.

Preferably, the device comprises equally spaced distance markers on the outer surface of the tube.
According to a second aspect of the present invention there is provided an implantation device for subcutaneous placement of a lead at a desired location, comprising:
a hollow tube having a proximal end and a distal end, the tube having a longitudinal opening extending from the proximal end to the distal end, the opening sized to receive the lead along the length of the opening, the distal end having a sharpened angled edge for penetrating living tissue, the edge having a U-shaped portion adapted for receiving one end of the lead; and
said lead comprising an elongated electrically conductive wire having a proximal end and a distal end, the distal end of the lead comprising an electrode having a 'fish hook" shaped contour adapted for retention thereof by the U-shaped edge portion of the hollow tube during placement of the lead electrode at the desired location.

Preferably, the hollow tube is formed of a biocompatible material.

Preferably, the hollow tube and the electrically conductive lead include spaced apart distance markers along their respective lengths for measuring the depth of insertion of the hollow tube and the lead below the surface of a patient's skin.

Preferably, the longitudinal opening is sized to provide at least for the removal of the lead therethrough upon completion of the placement of the lead electrode at the desired location.

Preferably, the lead comprises a wire having shape memory, the wire being flexible to provide for deformation thereof in a first state and returning to its memory in shape in a second state.

According to a third aspect of the present invention there is provided an implantation device for subcutaneous placement of a lead proximate to a neuromuscular pathway in living tissue, comprising:
a hollow tube having a proximal end and a distal end,
the tube having a longitudinal opening extending from the proximal end to the distal end,
the opening sized to receive the lead along the length of the opening,
the distal end having a sharpened angled edge for penetrating living tissue, and
said lead comprising an elongated electrically conductive wire having a proximal end and a distal end, the distal end of the wire having an electrode adapted for placement in contact with or adjacent to a desired neuromuscular pathway.

Preferably, the longitudinal opening extends essentially in a uniform straight direction between the proximal end and the distal end of the hollow tube.

Preferably, the lead comprises a wire having a shape memory, wherein the electrode has a memory in the shape of a wrap, the wrap dimensioned for encircling a desired neuromuscular pathway.

Preferably, the lead electrode is sufficiently flexible so as to be capable of being straightened in profile while within the hollow tube and to return to its memory in shape as the electrode is released from the hollow tube.

Preferably, the electrode comprises an electrical contact secured in an electrically insulating pad.

Preferably, the electrical contact comprises a plurality of individual electrical contacts, the lead electrically coupled to each one of the plurality of contacts.

Preferably, the electrical contact comprises a plurality of individual contacts, a different electrical lead coupled to each respective one of the plurality of contacts.

Preferably, the sharpened angled edge of the distal end of the hollow tube includes a U-shaped portion adapted to receive the lead electrode.

Preferably, the lead electrode has a "fish hook" shaped contour adapted for retention thereof by the U-shaped portion during placement of the lead electrode at the desired location.

According to a fourth aspect of the present invention there is provided a method of positioning a lead electrode proximate to or in contact with a desired neuromuscular pathway in living tissue, comprising the steps of:
(a) inserting an electrically conductive lead in a slotted elongated tube;
(b) positioning and maintaining the lead electrode at a distal end of the tube;
(c) inserting the tube and lead into living tissue to position the lead electrode adjacent to or in contact with a desired neuromuscular pathway;
(d) withdrawing the tube to a location external of said living tissue while maintaining the lead electrode in position adjacent to or in contact with a desired neuromuscular pathway; and
(e) sealing any surface tissue incision rendered in step (c) above.

Preferably, the method further comprises the steps of:
electrically stimulating the desired neuromuscular pathway with said electrode; and
monitoring the desired neuromuscular response to verify the location of said electrode with respect to the desired neuromuscular pathway.

Prerferably, step (d) further comprises the step of
releasing the lead from the tube through the slot.

According to a fifth aspect of the present invention there is provided a method of positioning a lead electrode proximate to or in contact with a desired neuromuscular pathway in living tissue comprising the steps of:
(a) inserting an electrically conductive metallic rod, having spaced apart distance markers, in living tissue to a depth location proximate to or in contact with a desired neuromuscular pathway;
(b) identifying a specific distance marker indicative of the depth of insertion of the metal rod;
(c) inserting an electrically conductive lead in a slotted elongated tube, said tube having a sharpened distal end and a plurality of equally spaced distance markers;
(d) positioning and maintaining the lead electrode at the distal end of the tube;
(e) placing the elongated tube and lead over the metallic rod;
(f) inserting the tube and lead into living tissue to a location adjacent to or in contact with, the desired neuromuscular pathway;
(g) removing the metallic rod;
(h) removing the elongated tube while maintaining the lead electrode in position adjacent to or in contact with, the desired neuromuscular pathway; and
(i) sealing any surface tissue incision rendered in steps (a) and (f).

Preferably, step (f) further comprises the step of
aligning the specific distance marker on the metallic rod with a corresponding distance marker on the elongated tube so as to position the lead electrode adjacent to or in contact with, the desired neuromuscular pathway.

Preferably, step (a) further comprises the steps of:
electrically stimulating the desired neuromuscular pathway with said rod; and
monitoring the neuromuscular response to verify the location of said rod with respect to the desired neuromuscular pathway.

Preferably, step (a) further comprises the steps of:
placing a dilator over the metallic rod,
inserting the dilator into living tissue to dilate the tissue surrounding the metallic rod; and
removing the dilator.

Preferably, step (h) further comprises the step of
releasing the lead from the elongated tube through the slot.

Preferably, step (h) further comprises the step of
electrically coupling the lead to a microstimulator/sensor.

Preferably, step (h) further comprises the step of electrically coupling the lead to an electrically conductive pad or coil.

For each of the aforementioned aspects of the present invention, any of the respective preferable features may be combined.

### BRIEF DESCRIPTION OF THE DRAWINGS

FIG. 1 is a perspective view of an example of an implantation device illustrating placement of a lead through a longitudinal opening in the device;

FIG. 2 depicts the device of FIG. 1 illustrating the lead positioned within the device;

FIG. 3 is a bottom view of the device of FIG. 1, illustrating placement of the lead electrode in a retaining recess;

FIG. 4 is a schematic illustration of a conductive needle inserted below the skin and positioned at a desired location and coupled to a stimulator/monitor;

FIG. 5 is a schematic illustration of a dilator placed over a needle positioned at a desired location;

FIG. 6 is a perspective view of the implantation device of FIG. 1 positioned above the skin prior to insertion and that is containing a needle positioned at a desired location;

FIG. 7 is a perspective view of the implantation device of FIG. 6 inserted to a desired location;

FIG. 8 is a schematic illustration of a lead positioned at a desired location and extending along and below the surface of the skin to a stimulator/monitor;

FIG. 9 is a schematic illustration of a lead electrode having a shape memory being wrapped around a neuromuscular pathway;

FIG. 10A is a schematic illustration of a lead having a shape memory electrode within the implantation of FIG. 1 and deformed from its original shape;

FIG. 10B is a schematic illustration of the electrode of the lead of FIG. 10A, partially extending beyond the distal end of the device;

FIG. 10C is a schematic illustration of the electrode of FIG. 10B further extending beyond the distal end of the device and wrapped around the neuromuscular pathway;

FIG. 11 is a perspective view of an electrode secured to an insulator; and

FIG. 12 is a perspective view of a plurality of electrodes recessed in an insulator.

### DETAILED DESCRIPTION OF THE INVENTION

The lead insertion tool of the present invention is used for subcutaneous insertion of the distal end of a wire lead to a desired location, typically adjacent or attached to a selected nerve, muscle, neuromuscular pathway or organ. Typically, the proximal end of the lead is attached to an implantable medical device, such as a microstimulator/sensor 28 as described for example in U.S. Patent No. 6,185,452 to Schulman, or an electrically conductive pad located on or below the surface of the skin. In this manner, electrical stimulation/sensing signals are communicated between the medical device or conductive pad and the distal end of the wire lead. The signals are to provide stimulation pulses to the neuromuscular pathway intended to actuate or motivate a target nerve, muscle or organ. In this manner, motor action of the target muscle may be induced. In the sensing mode, depolarization of the target muscle, which results in the release of electrical charge, may be sensed. Such signals are delivered, for example, during therapeutic activity sessions, body motion and command sessions and confirmation of desired placement of the distal end of the wire lead.

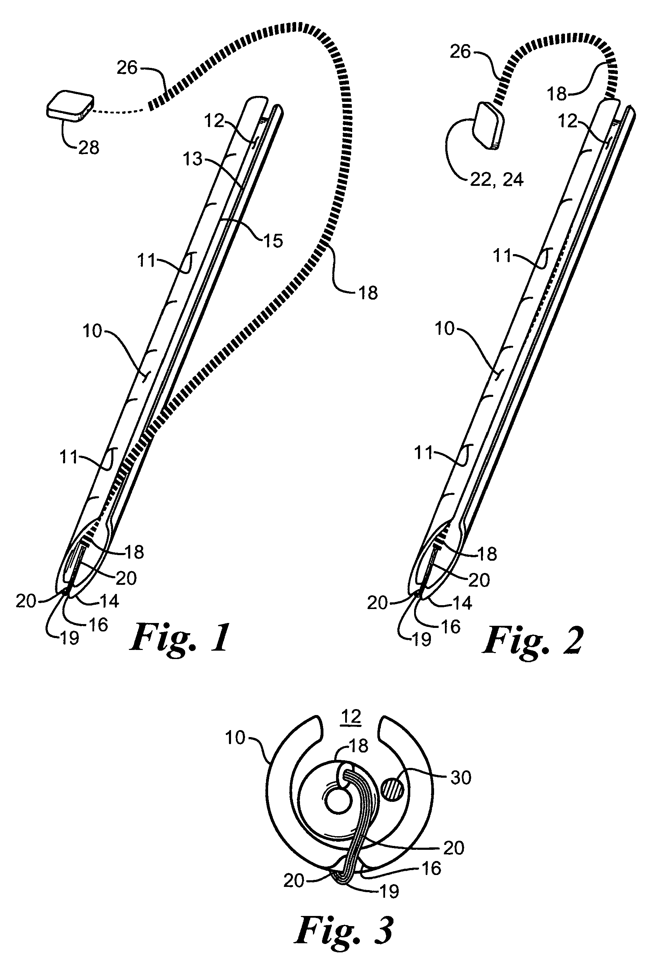

The insertion tool (FIG. 1) comprises an elongated tube 10 having an opening 12 extending the length of the tube 10. The edges 13 and 15 of the tube that define the opening 12 extend the length of the tube parallel to the tube's axis in a uniform straight direction. The tube 10 has a sharpened angled edge 14 at the insertion end of the tube. In one embodiment, a lead retaining contoured recess 16 is formed in the edge 14 which is adapted for retaining the distal end of lead 18 as the tube is inserted below the surface of the skin and for releasing the distal end of the lead as the tube 10 is being withdrawn. In an example configuration and to facilitate the retention action of recess 16, the lead 18 may have a "fish hook" type bend 19 that self locates in and is retained in place in recess 16. To accommodate contact with living tissue, the tube 10 is formed of a biocompatible material such as for example, titanium or stainless steel.

To insert lead 18 under the skin of a patient, the distal end of the lead, which typically includes an electrode 20, is either inserted lengthwise in the tube 10 or is inserted into tube 10 through opening 12. Alternatively, lead electrode 20 is positioned in recess 16 and the remainder of the lead may be inserted within the tube 10 through opening 12. The tube is positioned at a desired entry location on the patient's skin so that at the completion of the insertion procedure, electrode 20 will be positioned adjacent to or in contact with the desired neuromuscular pathway or (organ) 34 (hereinafter element 34) and the proximal end to a microstimulator/sensor 28 or electrically conductive pad 38.

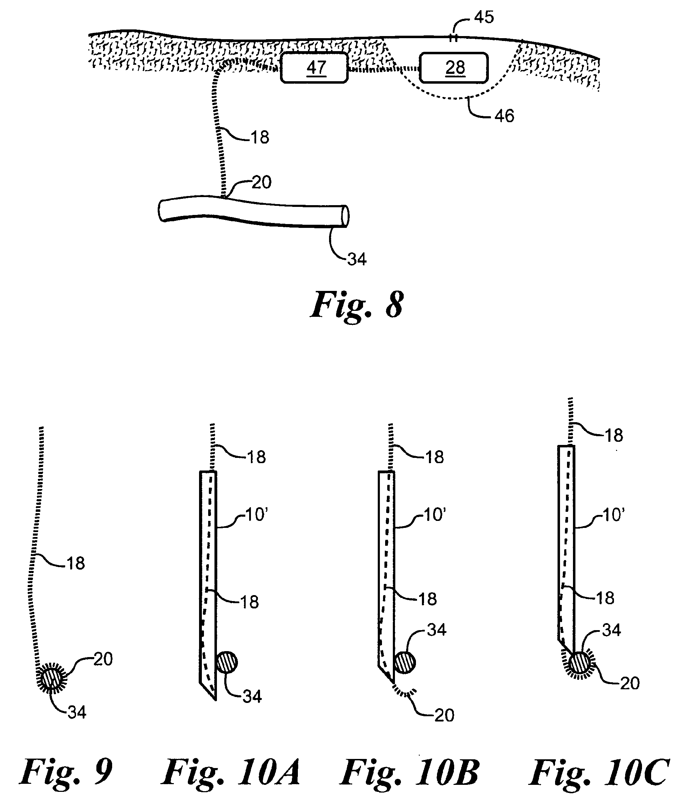

Upon application of force to the tube 10 against a patient's skin, the sharpened edge 14 punctures the skin or goes through a small slit made by a surgical knife and the tube 10 is inserted to a depth such that the electrode 20 is positioned at a desired location. The tube 10 can be retracted such that the electrode 20 remains in place and the tube 10 may be withdrawn such that the remainder of the lead exits the tube 10 through the opening 12. In this manner, as the tube 10 is withdrawn, lead 18 may be positioned just below and along the surface of the skin away from electrode 20 (see FIG. 8). The proximal end 26 of the lead 18 may be positioned at a location which facilitates receipt of signals from an external source which may be very difficult to achieve by electrode 20 with certain electrode 20 placements. Moreover the proximal end of the lead 18 may include either a coil 22 or pad 24 (FIG. 2) which receives/delivers signals from an external source. The proximal end 26 may also be attached to a leaded micro stimulator/sensor 28 (FIG. 8). The tube opening 12 provides for use of such coils and pads which are greater in dimension than the diameter of the tube 10. The opening 12 thus facilitates insertion of a lead having such pad or coil because the central portion of the lead 18 may be extracted from the tube 10 through opening 12 and thus the difficulty of passage of the pad or coil through the tube 10 is avoided. Subsequent to withdrawing and removing the tube 10, any incisions made in the skin of a patient may be sealed by suturing or other techniques known in the surgical arts.

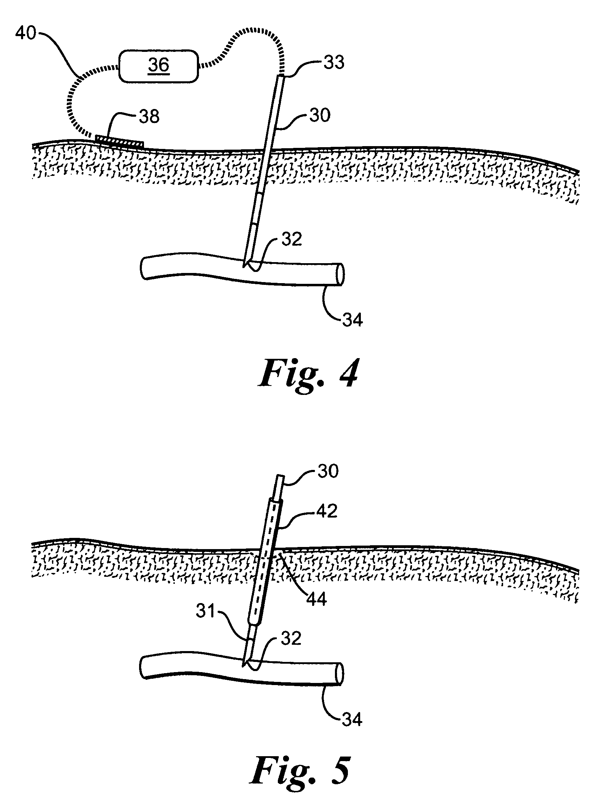

In practice, an electrically conductive needle 30 is inserted into and below the skin of a patient (see FIG. 4). The distal tip 32 is positioned adjacent a desired location on element 34. To confirm the needle positioning, a stimulation signal is delivered, by means of stimulator/monitor 36 (electrically attached to the proximal tip 33 of the needle 30) to element 34. The return path for the signal is through electrically conductive pad 38, which contacts the surface of the skin, and conductor 40, which is in electrical communication with stimulator/monitor 36. The needle 30 is electrically insulated along its length except for the proximal tip 32 and distal tip 33 which are exposed and therefore electrically conductive. In this manner, stimulation signals can be delivered and monitored without affecting tissue in contact with the needle 30 along its length.

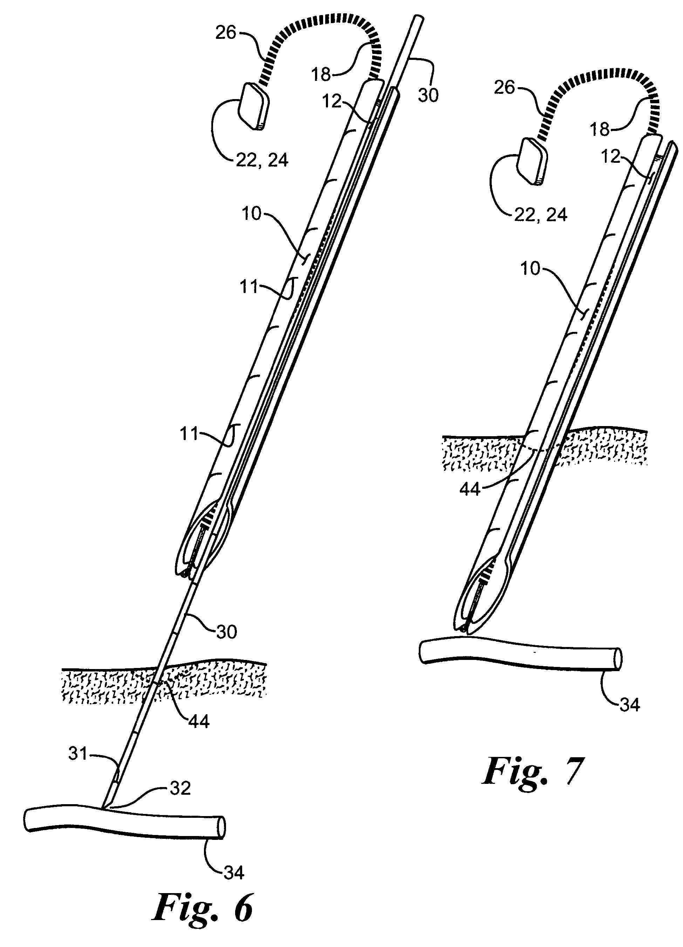

Upon confirmation that the desired element 34 reacts to an applied stimulation signal, a spreader or dilator 42 (see FIG. 5) is positioned over the needle 30 and inserted into and below the surface of the skin to spread apart tissue in anticipation of insertion of the lead insertion tube 10. The spreader 42 is inserted into the skin through a small slit 44 in the skin made by a surgical knife such as a scalpel. The slit 44 may be sealed in the manner described above.

The needle 30 has distance markers 31 evenly positioned along its length such that the depth of penetration of the needle below the skin is easily and visually determined. Upon removal of the spreader 42, the lead insertion tube 10 containing lead 18 is placed over the needle 30 (see FIG. 6) so that the needle acts as a guide, as the tube 10 is inserted through slit 44 until it reaches element 34. The depth of insertion of tube 10 is adjusted to match an observed distance marker of the needle 30 at the surface of the skin. The tube 10 includes distance markers 11 along its length and positioned at the same spacing as the distance markers on the needle 30. The depth of the insertion of the tube 10 can be matched to that of the needle by inserting the tube up to a marker consistent with that of needle 30. In this fashion, the depth of the tube 10 is such that the electrode 20 will thereby be positioned adjacent to or in contact with element 34. The inner dimension of tube 10 is selected to be large enough to accommodate both the lead 18 and needle 30 (see FIG. 3). Although the lead 18 is shown as a coiled conductor, it is within the contemplation of the present invention that the lead may comprise a rigid or semi-rigid straight single or multi-stranded conductor.

When the tube 10 is positioned adjacent the element 34 (see FIG. 7), the needle 30 is withdrawn and a stimulation signal is applied to lead 18 in a manner similar to the configuration shown in FIG. 4. Upon verification that the lead 18 is positioned as desired relative to element 34, due to the reaction of element 34 to the stimulation signal, tube 10 is withdrawn leaving behind the electrode 20 positioned as desired relative to element 34.

In those instances where a micro stimulator/sensor 28 is to be connected to lead 18 and implanted below the skin, a slit 45 is made into the skin to form a receiving "pouch" 46 into which is inserted the microstimulator/sensor 28 (see FIG. 8). The pouch 46 may also be used to contain a coil 22 or pad 24 as previously discussed. The pouch is then sealed or sutured closed. The slit 45, in a similar manner as provided with slit 44, may also provide the point of insertion of needle 30 and then tube 10 into the skin. The slit 45 is positioned at a convenient location which may be away from where electrode 20 is placed relative to element 34. Accordingly, as the tube 10 is withdrawn, it may be moved parallel to and just below the surface of the skin to a position in the vicinity of pouch 46. In this manner the lead will define a path from element 34 and then along and just below the surface of the skin to pouch 46. Furthermore, in the event lead 18 is to be electrically connected to a microstimulator/sensor 28 or pad 24, (coil 22), a "splice" connection 47 may be used to establish electrical communications between the lead and microstimulator/sensor 28 or pad 24 (coil 22). Although the splice connection 47 is shown in FIG. 8 external of the pouch 46, the connection 47 may also be located within the pouch 46.

In another example of the invention described herein, the lead 18 may be formed of an incompressible yet flexible metal structure such as a straight pin. In such an instance, by maintaining the pin position relative to the tube 10 constant, such as by gripping the pin and tube together, the pin need not have the fish hook shaped tip 19 nor does the tube require a recess to hold the lead 18. The tube 10 and pin 30 may be simultaneously inserted to the desired location and the tube 10 may thereafter be withdrawn leaving the lead 18 in place.

In yet another alternate embodiment, the electrode 20 may be configured to "wrap around", for example, element 34 at a specific location. The wrap around may be adjusted so as to make contact with the desired element 34 without undue squeezing which may lead to element damage. Advantageously, an electrically conductive elastic material having a preformed, essentially circular bend at its distal end may be used. Such elastic material is understood as a "shape memory alloy" or otherwise known as a "memory metal", which is a metal that "remembers" its geometry. The metal returns to its original geometry shape in one instance, during unloading from a geometry shape that has been temporarily altered from its original shape. The shape memory may also be accomplished by the use of a material that is preformed and then temporarily altered in shape below, however, the material's elastic limit.

More specifically and with reference to FIG. 9, the electrode 20 is shown wrapped around element 34. The electrode 20 grips element 34 sufficiently to insure satisfactory electrical contact with element 34 without causing any physical damage or injury thereto.

The steps undertaken to accomplish the gripping function are shown in Figs. 10A - 10C. In FIG. 10A, the tube 10 has been placed adjacent to, but beyond the element 34 a distance sufficient to be about the circumference of element 20 when it is wrapped around element 34. Preferably, lead 18 has an elastic circular profile that has essentially been "straightened out" or altered in shape when it is held within the tube 10. As the tube 10 is retracted, the electrode 20 exits the distal end of tube 10 and begins to take on its original circular profile, i. e. its memory shape, as is shown in FIG. 10B. As the tube 10 is further retracted or withdrawn, the electrode 20 begins to further exit the tube and curl around element 34 until the preformed portion of electrode 20 completely exits tube 10 and the electrode 20 returns to its original memory shape, while being wrapped around element 34. The remainder of lead 18 does not have a preformed circular shape and remains essentially straight, unless bent to a deliberate and desired contour during the lead insertion process.

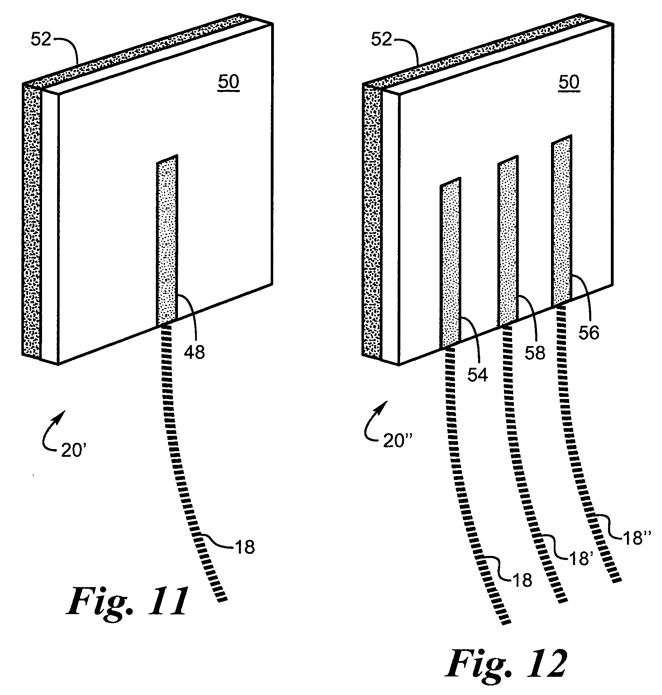

In those instances when an insulated electrode arrangement is desired, the embodiment of the electrode 20' shown in FIG. 11 is used. The electrode 20' comprises a biocompatible electrically conductive material 48 embedded in an electrically insulating material 50, that is backed by an insulator such as fabric or silicone rubber 52. The electrode 20' is preformed and has a memory shape as discussed above and forms a cuff around element 34 when released from tube 10 in the manner previously described. The insulating materials are sized and are sufficiently flexible to conform to the same contour as the memory shaped electrode. The lead 18 may be connected to a stimulator/sensor 28 in the manner shown in FIG. 8 or to a coil or pad as shown in FIG. 2.

In those instances when it is desirable to have multiple electrical contacts with an element 34, the example embodiment of FIG. 12 may be used. Although three contacts, i.e., 54, 56 and 58 are shown, it is to be understood that two or more contacts are contemplated by the invention, dependent upon functional requirements and size limitations. In this case, as in the embodiment of FIG. 11, the metal contacts 54, 56 and 58 have a memory shape and conforming insulation materials have characteristics as discussed above.

For the embodiment illustrated in FIG. 12, it is within the contemplation of the invention that a single lead may be electrically coupled to all contacts or that a different individual lead is coupled to a respective one of the contacts.

## Claims

1. An implantation device for subcutaneous placement of a lead proximate to a neuromuscular pathway in living tissue, comprising:
a hollow tube having a proximal end and a distal end,
the tube having a longitudinal opening extending from the proximal end to the distal end,
the opening sized to receive the lead along the length of the opening,
the distal end having a sharpened angled edge for penetrating living tissue, and
said lead comprising an elongated electrically conductive wire having a proximal end and a distal end, the distal end of the wire having an electrode adapted for placement in contact with or adjacent to a desired neuromuscular pathway.

2. The device of claim 1, wherein the longitudinal opening extends essentially in a uniform straight direction between the proximal end and the distal end of the hollow tube.

3. The device of claim 1 or 2, wherein the lead comprises a wire having a shape memory, wherein the electrode has a memory in the shape of a wrap, the wrap dimensioned for encircling a desired neuromuscular pathway.

4. The device of claim 3, wherein the lead electrode is sufficiently flexible so as to be capable of being straightened in profile while within the hollow tube and to return to its memory in shape as the electrode is released from the hollow tube.

5. The device of claim 1, 2, 3 or 4, wherein the electrode comprises an electrical contact secured in an electrically insulating pad.

6. The device of claim 5, wherein the electrical contact comprises a plurality of individual electrical contacts, the lead electrically coupled to each one of the plurality of contacts.

7. The device of claim 5, wherein the electrical contact comprises a plurality of individual contacts, a different electrical lead coupled to each respective one of the plurality of contacts.

8. The device of any one of claims 1 to 7, wherein the sharpened angled edge of the distal end of the hollow tube includes a U-shaped portion adapted to receive the lead electrode.

9. The device of claim 8, wherein the lead electrode has a "fish hook" shaped contour adapted for retention thereof by the U-shaped portion during placement of the lead electrode at the desired location.
